Europäisches Patentamt

(19) European Patent Office

Office européen des brevets

(11) Publication number: 0 027 518

A1

(12) EUROPEAN PATENT APPLICATION

(21) Application number: 80104986.7

(22) Date of filing: 21.08.80

(51) Int. Cl.³: A 61 F 9/06
G 02 F 1/133

(30) Priority: 06.09.79 US 72949
11.02.80 US 120675

(43) Date of publication of application:
29.04.81 Bulletin 81'17

(84) Designated Contracting States:
BE DE FR GB IT NL

(71) Applicant: GOR-VUE CORPORATION
29085 Solon Road
Cleveland Ohio 44139(US)

(72) Inventor: Gordon, Mack
1724 Wilbur Road
Medina Ohio 44256(US)

(72) Inventor: Harsch, Thomas B.
1908 Hibbard Drive
Stow Ohio 44224(US)

(72) Inventor: Fergason, James
5806 Horning Road
Kent Ohio 44240(US)

(74) Representative: Leiser, Gottfried, Dipl.-Ing.
Patentanwälte Prinz-Hauser-Leiser Ernsberger Strasse
19
D-8000 München 60(DE)

(54) Lens assembly for a welding helmet for use in sunlight conditions and in a welding helmet the combination of a circuit including a photodetector.

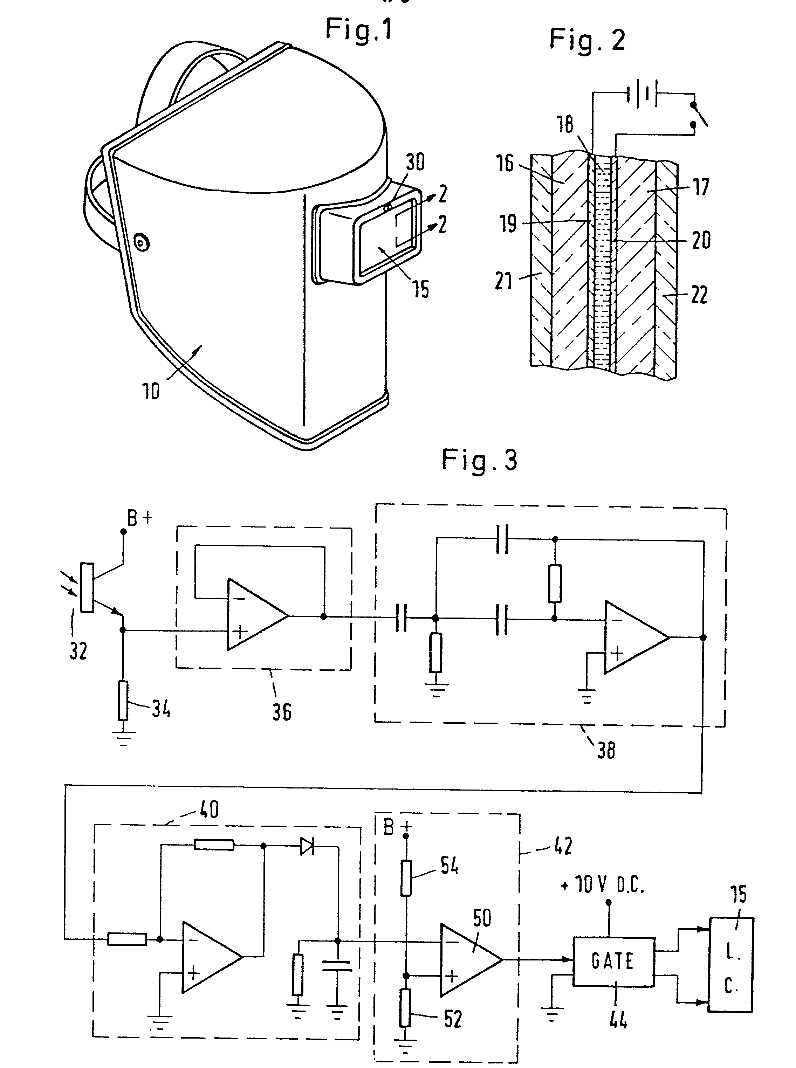

(57) A lens assembly is disclosed for use with a welding hel-
met. An optical lens is mounted on the helmet and exhibits a
normal first light transmissive condition and may be actuated
to a second eye protective, low light transmissive condition.
Circuitry, including a phototransistor, is provided for actuat-
ing the optical lens to its second condition only in response to
establishment of an electric arc between a welding electrode
and a workpiece, even during sunlight conditions, fluorescent
light conditions, and incandescent light conditions (Fig. 1).

Fig.1

## AN IMPROVED LENS ASSEMBLY FOR A WELDING HELMET
## FOR USE IN SUNLIGHT CONDITIONS

### Background and Field of the Invention

This invention relates to the art of welding helmets, and more particularly, to an improved protective welding lens assembly which may be used in sunlight conditions, fluorescent light conditions, as well as incandescent light conditions.

Although the invention will be described with particular reference to the use of an optical lens assembly employing a liquid crystal, it is to be appreciated that various other optical lenses may be employed in practicing the invention.

A protective lens assembly for a welding helmet which is known in the art is described in the United States Reissue Patent Re29,684, in the name of Mack Gordon. The lens assembly disclosed there includes an optical lens, which may take the form of a liquid crystal, and which exhibits a normal light transmissive state and which may be actuated to a low light transmissive state so as to protect the operator's eyes during welding operations.

The Gordon patent also discloses use of a photodetector responsive to light obtained from the establishment of an electric arc between a welding electrode and a workpiece to thereby actuate the optical lens from its light transmissive condition to its low light transmissive condition to thereby protect the eyes of the welder. This feature eliminates the need to raise or lower the helmet as is frequently done in welding operations. Instead, the operator, with the helmet down, may have clear visibility through the lens until the arc is ready to be struck. When the arc is struck, the lens will automatically darken to protect the eyes.

Whereas a helmet fitted with a lens assembly as described in the Gordon patent has found widespread use, it has, nevertheless, been normally limited to use in buildings which were protected from sunlight. This is because photodetectors being used respond to sunlight in a similar sense as that of an arc being struck at the welding electrode, and may cause the lens to darken prematurely. There is, then, a need for improvements in such a lens assembly such that an operator may perform welding operations in darkened rooms as well as in other light conditions, such as that of sunlight, incandescent light, or fluorescent light.

## Summary of the Invention

It is, therefore, a primary object of the present invention to provide improvements to the welding lens assembly described above so that the optical lens may be triggered by a welding arc even in environments in which the photosensitive means is exposed to sunlight or incandescent light or fluorescent light.

It is a still further object to provide photosensitive means for a welding lens assembly as described above wherein the photosensitive means is essentially nonresponsive to light emitted from fluorescent light sources, while passing light in the red portion of the visible spectrum to approximately 1.2 microns in the near infra-red region.

In accordance with the present invention, a lens assembly for a welding helmet has mounted thereon an optical lens. The lens has a normal first light transmissive condition and may be actuated to a second eye protective, low light transmissive condition. Circuitry, including photosensitive means, is provided for actuating the lens to its second condition only when the photosensitive means receives light from a welding arc even in conditions of exposure to sunlight or fluorescent light or incandescent light.

In accordance with a more limited aspect of the invention, photosensitive means is a semiconductor which is sensitive to light from the red portion of the visible spectrum to approximately 1.2 microns in the near infra-red region so as to thereby minimize its sensitivity to fluorescent light.

In accordance with another aspect of the invention, a protective automatic lens assembly is provided for welding helmets wherein the light-transmitting characteristics of the lens assembly are controlled by a photocell which senses only ultraviolet wave energy having a wavelength below about 3000 Angstrom units. As is known, the ozone above the atmosphere will absorb ultraviolet radiation in the 300 to 250 millimicron region; while the atmosphere itself will transmit this same band of radiation with little absorption. At the same time, the welding arc will emit wavelengths below 3000 Angstrom units, meaning that if a detector is provided which will sense ultra-violet wave energy in this band of wavelengths, the liquid crystal lens assembly can be used in direct sunlight and will be light-transmitting until a welding arc is established to emit the required wave energy of 3000 Angstrom units or less. Preferably, the photodetector is one having a cathode coated with cesium telluride which emits electrons in response to wave energy of 3000 Angstrom units or less.

Still further in accordance with the invention, the signal conditioning circuitry employs means for filtering out signals having frequencies indicative of received radiation from the sun or from an incandescent light source, while passing those indicative of radiation from a welding arc.

## Brief Description of the Drawings

The foregoing and other objects and advantages of the invention will become more readily apparent from the following description of the preferred embodiment of the invention as taken in conjunction with the accompanying drawings which are a part hereof and wherein:

Fig. 1 is a perspective view illustrating a welding helmet having a lens assembly in accordance with the present invention;

Fig. 2 is a fragmentary sectional view taken generally along line 2-2 of Fig. 1 and greatly enlarged;

Fig. 3 is a schematic-block diagram illustration of the improved circuitry for controlling operation of the lens assembly in accordance with the present invention;

Fig. 4 is a perspective view of a welding helmet incorporating a liquid crystal light shutter assembly with which the control system of the invention may be used;

Fig. 5 is a schematic circuit diagram of one embodiment of the invention utilizing a gated oscillator for driving a liquid crystal light shutter;

Fig. 6 is a schematic illustration of another embodiment of the invention wherein the liquid crystal cell is driven by a direct current voltage source;

Fig. 7 is a schematic circuit illustration of still another embodiment of the invention which utilizes a source of alternating current in combination with two ultraviolet-sensitive photocells; and,

Fig. 8 shows the spectral response characteristics of the photocell utilized in accordance with the invention.

## Description of Preferred Embodiment

Reference is now made to the drawings wherein the showings are for purposes of illustrating the preferred embodiment of the invention and not for purposes of limiting same. A welding helmet 10, which may be of conventional design, is illustrated in Fig. 1 and which includes an optical lens 15, positioned in the place of the usual eyepiece of such a helmet. The optical lens 15 acts as a light shutter in that upon being triggered, it will change state from a light-transmissive condition to a relatively non-light transmissive condition. In accordance with the invention, it should be understood that any convenient light shutter may be employed, providing it will respond to a trigger signal to be caused to change from such a light transmissive condition to an essentially non-light transmissive condition. A suitable light shutter may take the form of a liquid crystal light shutter as disclosed in the aforesaid patent to Mack Gordon, Re29,684, and the description thereof is incorporated herein. To facilitate an understanding of the present invention, the following background of such a light shutter will be presented, it being understood that a more complete description may be found in the Gordon patent.

Referring to Fig. 2, transparent plates 16 and 17, preferably of glass, are parallel and spaced apart by suitable spacers, not shown, by spacing of approximately 0.10 to 2.0 mils. The space between these plates is filled with a nematic-phase liquid crystal material 18 with a positive dielectric anisotropism. The molecules in a nematic-phase liquid crystal material appear to be long and straight, and they tend to lie parallel like matches in a match box. They are free to move with respect to one another and there is some variation with respect to exact parallelism. On the interior surfaces of the plates 16 and 17, and in contact with the liquid crystal layer 18, are coatings 19 and 20 of thin, transparent electroconductive material, known coatings being of tin oxide or indium oxide. Outside of the plates 16 and 17 are conventional polarizers 21 and 22, one of these layers being polarized at right angles to the other. In preparation of the liquid crystal sandwich, the layers 19 and 20 are prepared by being stroked or rubbed unidirectionally with, for example, a cotton cloth.

The effect of the liquid crystal sandwich 15 on light passing therethrough is discussed below. The transparent plates 16 and 17 are rubbed at right angles to each other such that a twisted nematic structure results in the liquid crystal material which will rotate plane-polarized light by 90°. However, when an electrical field is applied across the liquid crystal material by connecting the opposite terminals of a battery to the two transparent conducting films, the liquid crystal material will no longer rotate the plane of polarized light. Polarizers are disposed on either side of the cell. If the polarizers are parallel to each other, the liquid crystal light shutter will be essentially opaque in the absence of an applied electrical field (since the plane-polarized light is rotated through 90° in passing through the liquid crystal layer); however, when an electrical field is applied to the twisted nematic structure, the liquid crystal material will no longer rotate the plane of polarized light. Under these conditions, the liquid crystal cell will no longer be substantially opaque and can be seen through under ambient light conditions.

On the other hand, if the polarizers on opposite sides of the cell are crossed, then the cell will be light-transmitting until an electrical field is applied across the liquid crystal material; whereupon the cell will appear substantially opaque. Since the liquid crystal light shutter is substantially opaque in the presence of a welding arc, it has a light transmission of about 0.1%. Under these circumstances, the cell cannot be seen through under ambient light conditions; however, the intense light of a welding arc can be seen. When the liquid crystal cell is light-transmitting with the welding arc extinguished, it has a transmission of at least 6% whereby objects can be viewed through the cell under ambient light conditions.

Having now described a liquid crystal which may suitably
serve as the optical lens for this invention, attention is
now directed toward the circuitry employed for operating the
lens. As is brought out in the Gordon patent referred to herein,
it is known to employ photosensitive means mounted on the
helmet to respond to a welding arc together with amplifying
circuitry for triggering the optical lens from its normal
light-transmissive condition to its eye protective, essentially
opaque condition. As shown in Fig. 1 herein, a suitable aperture
30 is provided in the helmet in which there is mounted a photosensitive means to respond to the welding arc. As will be
described with reference to Fig. 3 herein, the photosensitive
means takes the form of a silicon phototransistor, together with
circuitry for gating the optical lens to its opaque condition
only in response to a welding arc, even though the arc is struck
in light conditions including sunlight, fluorescent light, or
incandescent light. These improvements permit an operator to
utilize such a helmet in operations whether indoors or outdoors.

Reference is now made to Fig. 3, which illustrates the improved
circuitry for actuating the optical lens 15 in response only to
light from a welding arc. The photosensitive means takes the
form of a silicon phototransistor 32 which is suitably mounted
in the aperture 30 on the welding helmet. This transistor has
its collector connected to a B+ potential (such as on the order
of +10 volts D.C.) and its emitter connected through a resistor 34 to ground. The transistor is sensitive only to light from approximately the red portion of the visible spectrum to about 1.2 microns in the near infra-red region. Consequently, the spectral response of the transistor minimizes its sensitivity to fluorescent light. As is known, fluorescent light tubes emit nearly all of their radiation in the visible region, and are particularly lacking in the red and near infra-red radiation levels. Such a transistor also responds rapidly to changes in light level over frequencies from approximately 0 to several hundred kilohertz.

Received light over the spectrum to which the transistor is sensitive will cause current to flow through the collector to the emitter circuit of the transistor, developing a potential across resistor 34. This is applied to a suitable amplifier 36 having unity gain and which may be conventionally comprised of an operational amplifier, as shown. The output of amplifier 36 is then passed by a multiple feedback, high pass filter 38, of conventional design, which serves to pass signals exhibiting a frequency on the order of 120 Hz and above. Signals below this frequency are strongly attenuated. It is known that incandescent lamps radiate most of their energy in the spectral region to which the silicon phototransistor responds. However, radiation from incandescent lamps is only weakly modulated at the rectifying line frequency, and is steady at all other frequencies. Sunlight, however, is a very strong source of radiation, which extends far beyond the spectral response region of the phototransistor. The sun is a very steady source of radiation, and contains virtually no modulation. Consequently, when the phototransistor 32 is exposed only to sunlight (no welding arc), the strength of any output signal provided by the high pass filter will be essentially zero.

Signals passed by the high pass filter are then amplified by an amplifier stage 40, of conventional design, with the amplified output signal then being supplied to a level detector 42. This level detector compares the strength of the amplified signal against a reference, and if the amplified signal is of sufficient magnitude, then a trigger signal will be supplied to turn on a suitable gate 44 to thereby de-energize the optical lens 15, causing the lens to be in its essentially opaque condition. The level detector 42 may include an operational amplifier 50 having a reference supplied to its positive input and the amplified signal supplied to its negative or inverting input. The reference signal may be obtained as from a potentiometer or from the junction of two resistors 52 and 54 connected in series between ground and a B+ potential. In the example being given, the level detector is set such that an output trigger signal is obtained only when the amplified output from amplifier 40 is on the order of 250 mv. This assures that signals passed because of exposure to incandescent light will not cause the trigger signal. As will be recalled, the high pass filter 38 will nevertheless pass signals of 120 Hz obtained from an incandescent lamp source, but these are only weakly modulated signals and are typically on the order of about 20 mv, well below the trigger level of detector 42.

The radiation of an electric welding arc is very intense. It has a spectral output from the deep infra-red to the far ultra-violet level. This radiation fluctuates slowly as the operator performs his work, and is strongly modulated at 120 Hz. This modulation is sufficient to cause a triggering effect for gate 44. The welding arc radiation is randomly modulated by noise from a frequency level of approximately 0 through tens of kilohertz. This modulation is detected by the circuitry herein over frequency levels from approximately 100 Hz to several kilohertz. These will be passed by the high pass filter 38 with essentially zero attenuation of those frequencies above 120 Hz. The output signal supplied by detector 42 to gate 44 effectively produces a clean digital signal which is used to control actuation of the liquid crystal 15. If desired, the circuitry comprising gate 44 may take the form of the circuitry already disclosed in the aforementioned patent to Mack Gordon, Re29,684.

From the foregoing, then, it is seen that the present invention provides an effective manner of controlling an optical lens so as to be actuated from its normal light transparent condition to an essentially opaque condition in response only to light emanating from a welding arc, even though the operator may be located so as to be exposed to sunlight or to fluorescent light or to incandescent light.

In accordance with another aspect of the present invention, a photocell is employed which is responsive only to ultraviolet wave energy having a wavelength less than about 3000 Angstrom units. Ultraviolet wave energy from the sun in this energy band is filtered by the ozone above the atmosphere; while the atmosphere itself will transmit ultraviolet wave energy in this range with little absorption. In this regard, the attenuation of ultraviolet wave energy in the range of about 2500 to 3000 Angstrom units at sea level is only 70% in 2000 yards.

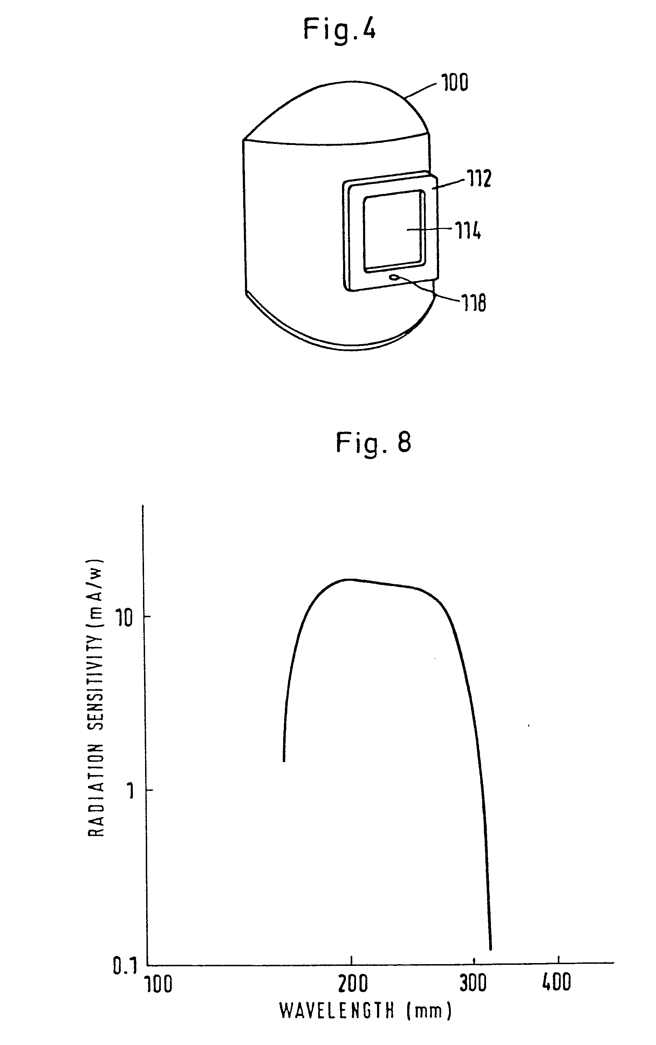

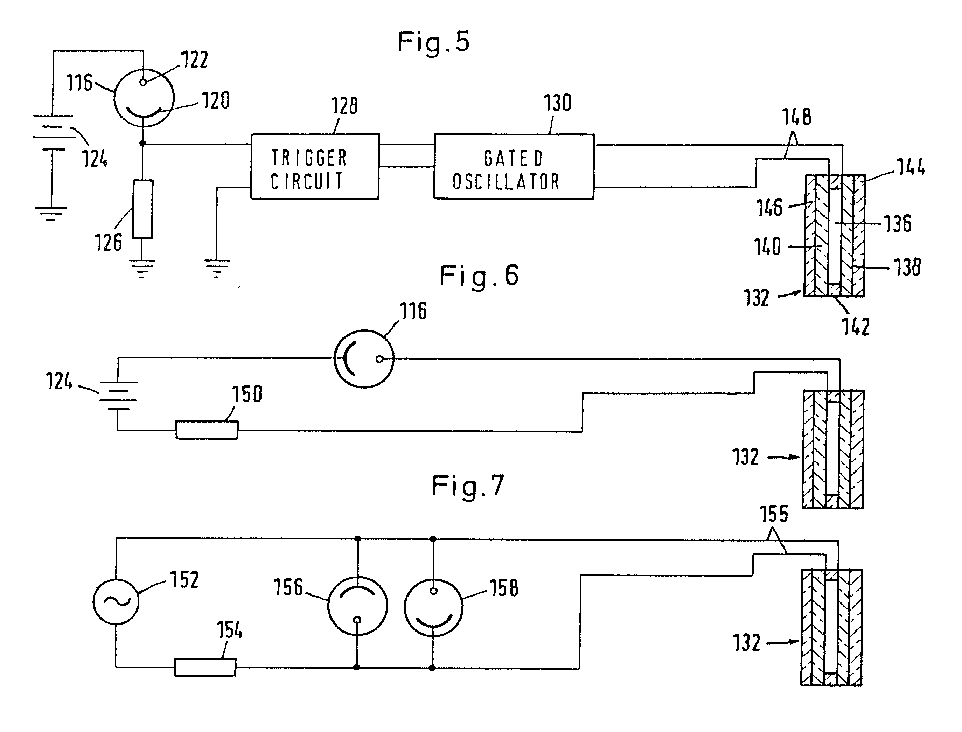

With specific reference to Fig. 5, the photocell utilized in accordance with the invention is identified by the reference numeral 116. This is positioned behind or within an aperture 118 in the welding helmet lens assembly 112 shown in Fig. 4, along with a light shutter 114 on a helmet 110. The photocell 116 includes a cathode 120, which is exposed to incident light, and anode 122. Cathode 120 is coated with a layer of cesium telluride which will emit electrons in response to ultraviolet wave energy in the 150 to 300 millimicron range. This is shown in Fig. 8 wherein the curve represents the spectral response of cesium telluride. Thus, when (and only when) ultraviolet wave energy beneath 3000 Angstrom units falls upon the cesium-telluride coated cathode 120, the cathode will emit electrons and the photodiode will act as an essentially closed switch.

Diode 116 has its anode 122 connected to the positive terminal of a battery 124; while the cathode 120 is connected through a resistor 126 to ground and, hence, to the cathode of battery 124. When ultraviolet wave energy having a wavelength beneath 3000 Angstrom units falls on the cathode 120, the diode 116 conducts to raise the voltage at the upper end of resistor 126. This rise in voltage is sensed by a Schmidt trigger circuit 128 which is essentially a voltage comparator having a high input impedance, a low output impedance and a nearly discontinuous transfer characteristic. Its output changes abruptly from a low or high state to a high or low state, respectively, as the input voltage at the upper end of resistor 126 rises above a predetermined threshold voltage. The threshold of such devices is commonly one-half of the supply voltage. The output can be either inverting or non-inverting. The gated oscillator 130 will be ON and will be turned OFF in response to a rise in voltage across resistor 126 when parallel polarizers are used on the liquid crystal cell 132; however, it will be turned ON in response to the same rise in voltage across resistor 126 in the case of crossed polarizers.

The liquid crystal cell itself is identified in Fig. 5 by the reference numeral 132. It comprises a layer of nematic liquid crystal material 136 disposed between transparent parallel plates 138 and 140 which, as mentioned above, are rubbed at right angles to each other. A gasket 142 disposed beneath the plates 138 and 140 surrounds the liquid crystal layer 136. On either side of the plates 138 and 140 are polarizers 144 and 146 which may be crossed or parallel as explained above, depending upon the operation of the unit. The facing surfaces of the transparent plates 138 and 140 are coated with transparent conductive material. These layers of conductive material are connected through leads 148 to the output of gated oscillator 130 which applies an electric field across the liquid crystal layer.

In Fig. 6, another embodiment of the invention is shown wherein elements corresponding to those of Fig. 5 are identified by like reference numerals. In this case, however, the photocell 116 is connected in series with the battery 124 and a resistor 150 across the transparent conducting films of the liquid crystal cell 132. In the absence of ultraviolet wave energy of wavelength beneath 3000 Angstrom units, the photocell 116 will act as an open switch. However, when wave energy below 3000 Angstrom units is sensed by the photocell, it will act as a closed switch, thereby connecting the battery 124 across the transparent conductive films of the liquid crystal cell 132. In the embodiment of Fig. 6, it will be appreciated that the polarizers will be crossed such that the cell will be light-transmitting until ultraviolet wave energy from the welding arc is sensed.

In Fig. 7, another embodiment of the invention is shown wherein elements corresponding to those of Fig. 3 are again identified by like reference numerals. In this case, however, the liquid crystal light shutter 132 will have parallel polarizers such that it will be light-transmitting only when an electric field exists across its transparent conductive coatings. This electric field is supplied by an alternating current source 152 which is connected through resistor 154 and leads 155 to the transparent conductive films. Connected in shunt with the alternating current source 152 and the resistor 154 are two reverse-polarity photocells 156 and 158 which, as in the previous embodiments, will conduct only in response to ultraviolet wave energy having a wavelength below about 3000 Angstrom units. When such wave energy appears upon the existence of a welding arc, the two photocells 156 and 158 will effectively short out the current source 152 to remove the electric field from the transparent conductive films of the liquid crystal light shutter 132, whereupon it becomes opaque.

In all of the foregoing cases, ordinary sunlight will not affect the photocells since, as explained above, the ozone above the atmosphere filters out ultraviolet wave energy having a wavelength less than about 3000 Angstrom units.

Although the invention has been described in conjunction with a preferred embodiment, it is to be appreciated that various modifications and arrangements in parts may be made within the spirit and scope of the invention as defined by the appended claims.

0027518

-1-

What is claimed is:

1. A lens assembly for a welding helmet, comprising
optical lens means having a normal first light
transmissive condition and actuatable to a second light
transmissive condition; and
circuit means, including photosensitive means,
for actuating said lens means to its second condition in response
to light received by said photosensitive means from a welding
arc, even in conditions in which said photosensitive means is
exposed to sunlight or fluorescent light or incandescent light.

2. A lens assembly as set forth in claim 1, wherein said
photosensitive means includes means responsive to light in the
red portion of the visible spectrum through the near infra-red
region.

3. A lens assembly as set forth in claim 1, wherein said
photosensitive means is essentially non-responsive to fluorescent
light.

4. A lens assembly as set forth in claim 1, wherein said
photosensitive means includes means responsive to light in the
red portion of the visible spectrum to approximately 1.2 microns
in the near infra-red region.

5.  A lens assembly as set forth in claim 1, wherein said photosensitive means provides an output signal in dependence upon light received.

6.  A lens assembly as set forth in claim 5 wherein said circuit means includes trigger means for providing a trigger signal for actuating said lens means and signal conditioning means for actuating said trigger means only when said output signal has a value indicative that the received light included light from a welding arc.

7.  A lens assembly as set forth in claim 6, wherein said output signal may have a variable value over a frequency range and/or a variable amplitude and said signal conditioning means includes means responsive to both frequency and amplitude of said output signal for actuating said trigger means.

8.  A lens assembly as set forth in claim 7, wherein said signal conditioning means includes means for filtering out those said output signals having frequencies indicative of received radiation from sources other than a said welding arc.

9.  A lens assembly as set forth in claim 8, wherein said signal conditioning means further includes amplitude responsive means for responding to filtered output signals exhibiting a predetermined magnitude representative of radiation from a said welding arc for actuating said trigger means.

10. In a welding helmet of the type employing a liquid crystal light shutter as a lens assembly, the combination of a circuit including a photodetector exposed to light emitted from a welding arc and responsive only to light having a wavelength less than about 300 millimicrons for causing said light shutter to increase in opacity when said light of wavelength less than about 300 millimicrons is emitted by a welding arc.

11. The combination of claim 10 wherein said liquid crystal light shutter comprises a layer of liquid crystal material sandwiched between transparent conductive films, a gated oscillator connected to said transparent conductive films, trigger circuit means for controlling said gated oscillator, and means including said photodetector for controlling said trigger circuit.

12. The combination of claim 10 wherein said liquid crystal light shutter comprises a layer of liquid crystal material sandwiched between transparent conductive films, a source of direct current potential, and means including said photodetector for applying said source of direct current potential across said transparent conductive films only when the photodetector senses ultraviolet wave energy having a wavelength less than about 300 millimicrons.

13. The combination of claim 10 wherein said liquid crystal light shutter comprises a layer of liquid crystal material sandwiched between transparent electrical conducting films, a source of alternating current potential applied across said transparent conducting films, and means including a pair of reverse-polarity photodetectors actuable in response to light of wavelength less than 300 millimicrons in shunt with said source of alternating current voltage.

14. The combination of claim 10 wherein said photodetector includes a cathode coated with cesium telluride.

0027518

Fig.1

Fig. 2

Fig. 3

# Fig.4

# Fig. 8

Fig.5

Fig.6

Fig.7

European Patent Office

**EUROPEAN SEARCH REPORT**

| | DOCUMENTS CONSIDERED TO BE RELEVANT | | CLASSIFICATION OF THE APPLICATION (Int Cl. ¹) |
|---|---|---|---|
| Category | Citation of document with indication, where appropriate, of relevant passages | Relevant to claim | |
| A | <u>DE - B - 1 491 201</u> (THE NATIONAL) <br> + Column 1, lines 57-60; column 2, lines 4-10, 65, 66; column 3, line 7; claim 1 + <br> -- | 1-4 | A 61 F 9/06 <br> G 02 F 1/133 |
| D,A | <u>US - E - 29 684</u> (MACK GORDON) <br> + Column 3, lines 36-62; column 4, lines 55-68; column 5, lines 1-9; fig. 2, 11, 12 + <br> -- | 1-6, 10-12 | |
| A | <u>DE - A - 2 355 889</u> (NATIONAL RESEARCH) <br> + Page 16, lines 5-8; page 17, lines 15-19; claim 1; fig. 3, 4 + <br> -- | 1,5, 11-13 | TECHNICAL FIELDS SEARCHED (Int.Cl. ³) <br><br> A 61 F 9/00 <br> G 02 C 7/00 <br> G 02 F 1/00 |
| A | <u>DE - B - 1 952 023</u> (RCA CORPORATION) <br> + Column 3, line 57 - column 4, line 44; fig. 3 + <br> -- | 1-6,8, 10-12 | G C9 F 9/00 <br> H 01 J 1/00 <br> H 01 J 9/00 <br> H 01 J 40/00 |
| A | <u>DE - A - 2 149 661</u> (WESTERN ELECTRIC) <br> + Page 5, lines 15-21; fig. 4 + <br> -- | 1-6,10, 11 | H 04 N 5/00 |
| A | <u>US - A - 4 130 903</u> (TECHN. BUREAU A.B.O.F) <br> + Column 2, lines 22-28, 63-68; column 5, lines 24-42; column 7, lines 16-18, 34-43; fig. 3-5 + <br> -- | 2,4,5, 8,9 | CATEGORY OF CITED DOCUMENTS <br><br> X: particularly relevant <br> A: technological background <br> O: non-written disclosure <br> P: intermediate document <br> T: theory or principle underlying the invention <br> E: conflicting application <br> D: document cited in the application <br> L: citation for other reasons |
| A | <u>US - A - 4 071 912</u> (REVUE THOMMEN AG) <br> + Column 2, lines 62-66; column 3, lines 29-46; fig. 4 + <br> -- | 3,5, 10-13 | &: member of the same patent family, corresponding document |
| X | The present search report has been drawn up for all claims | | |

| Place of search | Date of completion of the search | Examiner |
|---|---|---|
| VIENNA | 19-11-1980 | TROJAN |

EPO Form 1503.1 06.78

**European Patent Office**

## EUROPEAN SEARCH REPORT

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| | AT - B - 150 578 (PATENT-TREUHAND-GESELLSCHAFT) <br> + Lines 7-20 + <br> -- | 14 | |
| | DE - B - 1 078 702 (ELECTRIC & MUSICAL) <br> + Column 1, lines 36-41; column 4, line 4; claims 3,8 + <br> ---- | 14 | TECHNICAL FIELDS SEARCHED (Int. Cl.³) |